Europäisches Patentamt

European Patent Office

Office européen des brevets

(11) Publication number: **0 262 942**
**A1**

## (12) EUROPEAN PATENT APPLICATION

(21) Application number: 87308646.6

(22) Date of filing: 29.09.87

(51) Int. Cl.⁴: **C 12 N 15/00**
C 12 N 5/00

(30) Priority: 30.09.86 US 913822

(43) Date of publication of application:
06.04.88 Bulletin 88/14

(84) Designated Contracting States: **ES GR**

(71) Applicant: **SMITHKLINE BECKMAN CORPORATION**
One Franklin Plaza P O Box 7929
Philadelphia Pennsylvania 19103 (US)

(72) Inventor: **Rosenberg, Martin**
241 Mingo Road
Royersford Pennsylvania 19468 (US)

**Sweet, Raymond Whitney**
108 Edgehill Road
Bala Cynwyd Pennsylvania 19004 (US)

(74) Representative: **Giddings, Peter John, Dr. et al**
Smith Kline & French Laboratories Ltd. Corporate
Patents Mundells
Welwyn Garden City Hertfordshire AL7 1EY (GB)

(54) **Cell transfection.**

(57) A recombinant mammalian cell line is prepared by cotransfection of a cell with a gene of interest and a protooncogene.

EP 0 262 942 A1

**Description**

Cell Transfection

FIELD OF THE INVENTION

This invention relates to a method for transfecting eukaryotic cells and transfectant cell lines produced thereby.

BACKGROUND OF THE INVENTION

Mammalian cells are the hosts of choice for production of many gene products by recombinant DNA procedures. Various techniques exist for introducing a gene of interest into mammalian cells. These, include, generally, cotransfection of the gene of interest with a selection marker. Typically the selection marker is a gene which confers resistance to a certain drug or ability to utilize a certain nutrient. Techniques for producing cells having multiple copies of a gene of interest integrated into the host cell chromosomal DNA include amplification by selection pressure. For example:

Axel et al., U.S. 4,399,216, disclose amplification of methotrexate (MTX) sensitive Chinese hamster ovary cells (CHO) by cotransfection with a gene for dihydrofolate reductase (DHFR).

Wahl et al., PCT WO82-158 report a similar procedure involving a gene for resistance to N-(phosphonoacetyl)-L-aspartate (PALA), the CAD gene.

Such amplification techniques require serial passages in increasing concentrations of the selective agent in a process that can require months to obtain high copy number. The selection pressure generally must be maintained to insure stability of the cell line.

Amplification of protooncogenes has been associated with transformation. Oncogenes are genes which function in the process of transformation of a normal cell to a malignant state. Protooncogenes are normal cellular genes which become oncogenic upon either mutation or aberrant expression.

Varmus, Ann. Rev. Genetics 18:553 (1984), reviews cellular oncogenes. At page 587, Varmus reviews structural features of amplified oncogenes, including that amplified domains can be "100 to 1000 kb or more," suggesting that "adjacent genes may be co-amplified."

DeFeo et al., Proc. Natl. Acad. Sci. USA 78:3328 (1981), and Chang et al., Nature 297:479(1982), report that established rodent fibroblast cell lines, such as NIH 3T3, can be growth transformed at low efficiency by the introduction of normal mammalian ras genes.

Pulciani et al., Molec. Cell. Biol. 5:2836(1985), report that amplification of the H-ras protooncogene is associated with malignant transformation of NIH 3T3 cells.

SUMMARY OF THE INVENTION

This invention lies in the discovery of a technique for transfecting cells and selecting multicopy transfectants, in a single transfection and selection procedure. More specifically, the invention is a method for producing a recombinant mammalian cell line having multiple copies of a gene of interest which comprises: (1) cointroducing into a mammalian cell, which can be transformed by increased expression of a protooncogene, the gene of interest and the protooncogene and (2) selecting transfectants which are transformed and which express the gene of interest.

In another specific embodiment, the invention is a recombinant mammalian cell prepared by the method of this invention.

These and other embodiments are considered further aspects of the same invention which is fully disclosed herein below.

DETAILED DESCRIPTION OF THE INVENTION

The present invention provides a means of transfecting a mammalian cell with a gene of interest and rapidly selecting transfectants having multiple integrated copies of the gene of interest. By the method of the invention, recombinant cells having up to 500 or more copies of the gene of interest can be manufactured in a matter of days. By way of comparison, transfection by conventional techniques involving cotransfection with a selection marker, e.g., DHFR or G418 or hygromycin resistance, usually results in initial integration of no more than a few copies of a gene of interest, e.g., 1 to 10. Amplification to comparable gene copy number by selection pressure amplification techniques, e.g., the DHFR/MTX technique, can require several months to accomplish. Furthermore, the present invention does not necessarily require selection pressure, although use of selection pressure as an adjunct to this method of the invention is, of course, not precluded.

Several protooncogenes which function as oncogenes upon increased expression can be used in this invention, are known. These, include normal mos, sis, abl, myb, src and myc genes. Cells which can be growth transformed by transfection with these genes and by abormal, i.e., elevated, expression of the gene products are also known. Such cells are contact inhibited prior to transfection in high copy number and are transformed following transfection in high copy number. Transformation is manifested by observable phenotypic change caused by expression of the oncogene product, such as change in growth, morphology, or biochemical properties e.g., by immortalization, by foci formation in monolayers, by growth in soft agar, growth in reduced serum, or by tumorigenicity in vivo.

The preferred protooncogene is a normal ras gene. The ras gene family includes the H-ras, K-ras and N-ras genes. All of these code for a transforming protein, p21, of 21 to 24 kilodaltons. All exist as protooncogenes, or normal genes, which confer the transformed phenotype upon increased expression or upon mutation. The normal H-ras gene is especially preferred.

For cotransfection with a normal H-ras gene, i.e., a H-ras protooncogene, a contact inhibited rat fibroblast cell line, especially the NIH 3T3 cell line is preferred. Other useful cell lines include the Normal rat kidney (NRK) (ATCC 1571) cell line, the rat embryo fibroblast 52 (REF-52) cell line (McClure et al., Cold Spring Harbor Conferences on Cell Proliferation 9:345-364(1982)) and other contact inhibited cell lines.

The gene of interest can be any coding sequence operatively linked to regulartory functions required or desirable for transcription and translation of the coding sequence. Regulatory functions include, for example, functions required for RNA-polymerase binding, transcription initiation, ribosome binding and translation, as well as other functions such as polyadenylation and enhancement transcriptional sequences. The promoter can be regulatable so that, for example, expression is not induced until after transfection and selection of transformed clones. The gene of interest can be, for example, one which codes for a hormone, a lymphokine or other pharmaceutically active agents such as serum or tissue proteins, e.g., tissue plasminogen activator and urokinase.

Transfection is carried out by standard techniques. See, for example, Wigler et al., Proc. Natl. Acad. Sci. USA 76:1373 (1979) and Copeland et al., Cell 17:993 (1979). These techniques include, for example, calcium phosphate precipitation, DEAE-dextran induced pinocytosis, electroporation and viral transfection. The cells are transfected with a normal ras gene and with a gene of interest. The two functions can be linked, i.e., carried on the same DNA molecule, or they can be unlinked, i.e., on different DNA molecules. Greater efficiency may be obtained when both the gene of interest and the protooncogene are carried on the same DNA molecule.

Following transfection and selection of transformed cells, transformed cells are cloned by standard techniques. Stable transformed cells are then selected for analysis. Clones which express high levels of the gene of interest are selected for further culturing. Such cells are cultured and the product of interest is collected and isolated in accordance with standard techniques.

In carrying out the method of the invention transformed cells may be selected in which transformation is due to mutation of the normal gene to an activate or mutant oncogene. Such event, which will occur at low frequency at most, will be readily identified by failure of the transformed cells to express large amounts of the gene of interest relative to other transformed cells or by genetic or biochemical analysis.

Modification of the method of the invention to improve efficiency or to tailor a cell line to suit a particular need can be made. For example, the protooncogene can be placed under control of a regulatable promoter. In this way, transformed cells selected under optiomal expression of the protooncogene can be reverted to a normal phenotype by turning off the promoter after transfection and selection.

In cases of transfection with selection markers requiring selection pressure, transfected DNA sequences may remain fixed in the genome with little rearrangment after more than 100 population doublings, under selection pressure. In the absence of selection pressure, cells in which the transfected sequences are lost or rearranged accumulate at variable rates. In another modification of this invention, cointroduction of a second selectable marker, such as G418 resistance, provides a partial alternative to identifying conditions selective for the protooncogene transformed cells. This approach also simplifies the clonal isolation of the growth transformed cells eliminating the normal cells removed from the monolayer with foci. Because this second marker is only physically – not functionally - associated with the multiple copies of the protooncogene, it cannot protect against specific loss of the protooncogene. However, this approach can maintain a more homogenous transfected cell population. Although growth transformation is selectable by focus formation, we have not identified maintenance conditions which will select against non-transformed cells arising in the culture. Chiang et al., In Vitro Cell Devel. Biol. 21:707 (1985), define serum-free conditions favoring the growth of NIH 3T3 cells transformed by the T24 ras gene, i.e., the mutant oncogene, relative to NIH 3T3 cells themselves. These conditions may also be applicable to cells transformed by overexpression of the normal ras gene. However, these conditions may not be selective against NIH3T3 cells in mixed culture with the ras-transformed cells because secreted products of the transformed cells may support the growth of the normal cells.

## EXAMPLES

The Examples which follow are illustrative, and not limiting, of the invention. Restriction endonucleases and other enzymes used in the genetic manipulations were obtained from commercial sources and were used substantially in accordance with the vendors' instructions. Except where otherwise indicated, procedures were carried out substantially as described by Manatis et al., Molecular Cloning, Cold Spring Harbor Laboratory, 1982. All temperatures are in °C.

## A. Vectors:

The human H-ras coding sequence including 3′ untranslated sequences from pUC69W (Gross. et al., Mol. Cell. Biol. 5:1015 (1985) was inserted into an expression vector under the transcriptional control of the SV-40 early promoter to prepare a vector identified as pMERcH-ras. The fragment from pUC69W was a blunt-ended fragment prepared by digestion with Sal I followed by treatment with mung bean nuclease and then digestion with NdeI followed by filling in with DNA polymerase I (Klenow).

pMERT24 was prepared by exchanging the val-12 mutant H-ras coding sequence for the normal ras fragment in pMERcHras. The mutant oncogene was excised from pUC69 (Gross et al., above) by digestion with HindIII and ApaI.

pRas-neo and pT24-neo were prepared by inserting a 1.5 kb BamHI fragment containing the neomycin resistance (neoR)cassette from pSV2neo (Southern et al., J. Molec. App. Genet. 1:327 (1982) into pMERcHras and pMERT24, respectively, in the same transcriptional orientation as the ras genes.

pTPA-25 is a vector having a tissue plasminogen activator (tPA) coding sequence on a SalI cassette between the SV 40 early promoter and polyadenylation signals.

pDSP1.1TPA25BGH is a vector having a tissue plasminogen activator (tPA) coding sequence on a SalI cassette under control of the SV 40 early promoter and the bovine growth hormone (BGH) polyadenylation region inserted into pDSP1 (Pfarr et al., DNA 4:461 (1985)). A BGH polyadenylation region is disclosed, for example, by Woychik et al. Biochem, 81:3944 (1984) and by Goodwin et al., EP-A-173,552,.

pRDZBTPA was prepared by replacing the SV-40 promotor/enhancer element in PDSP1.1TPA25BGH with an a Rous sarcoma virus (RSV) LTR.

pRasTPA plasmids: pRasTPA-13 was constructed by insertion of a tPA transcription cassette into a SalI site of pMERcHras. The tPA cassette was excised from pDSP1.1TPA25DBGH on a 3046 bp SalI fragment- pRasTPA38 was similarly constructed with a 2957 bp Sal I tPA cassette from pRDZBTPA. In both of these ras-TPA vectors, the ras and tPA genes have the same transcriptional orientation.

B. Cell Culture:

NIH 3T3 cells were maintained in Dulbecco's minimal essential medium (DMEM) (GIBCO, Grand Island, New York) with 1.85 g/l NaHC03, 100 units/ml penicillin and streptomycin and 10% Nu-serum (Collaborative Research Cambridge, Massachusetts) in 5% CO2 NIH 3T3 cells were seeded from frozen stocks and were carried at subconfluence for about 2 months and then discarded. Cells transformed by the ras gene were selected and maintained in DMEM/5% Nu-serum.

C. DNA Transfection:

DNA transfections were carried out as described by Wigler et al., above, or by Copeland et al., above. 10 ug of calcium-phosphate precipitated DNA was added to about $10^6$ NIH 3T3 cells in a 60 mm dish. The media was changed 5-8 hrs later. For focus selection, the cells were split 1:3 into 100 mm dishes in DMEM/5% Nu-serum about 24 hours after transfection. For selection of G418 resistance, the cells were split 1:10-40 into DMEM/10% Nu-serum containing 600 ug/ml G418. The selective media were changed every 3-4 days.

D. Nucleic acid isolation and hybridization:

Preparation of plasmid and cellular DNA and DNA blotting were substantially as described or by Deen et al., J. Virol. 57:422 (1986), according to Maniatis et al, above. Cellular RNA was isolated essentially as described by Chirgwin et al., Biochem. 18:5294 (1979). Attached cells in culture were rinsed with PBS and lysed in citrate-buffered guanidinium thiocyanate, pH 7.0 Cellular RNA was then separated from other constituents by centrifugation through 5.7 M CsCl for 20 hours at 42°. For RNA blots, samples were electrophoresed on formaldehyde gels and transferred to nitrocellulose. Filters were prehybridized in 6 × SSPE (1× = 10 mM NaH2P04, 1 mM EDTA, 180 mM NaCl, pH 7.4), 50% formamide, 200 ug/ml yeast RNA and 5 × Denhardts' solution (Denhardt, Biochem. Biophys. Res. Comm. 23:614 (1966). Hybridizations were for 48 hrs in the same buffer with the addition of 32P-labelled robes ($10^6$ cpm/ml; 1-4 × $10^8$ cpm/ug), prepared by nick translation. The filters were then washed three times for 10 min in 2 × SSPE-0.1% SDS room temperature and three times for 10 min in 0.1 × SSPE-0.1% SDS at 55°. For quantitation of DNA blots, plasmid DNA cleaved with the appropriate restriction enzyme was included on each blot. For some RNA blots, plasmid or specific coding fragments were slot-blotted onto nitrocellulose strips and hybridized in parallel.

E. Protein preparation and immunoblot analysis:

Cells were scraped from culture dishes in phosphate buffered saline (PBS), collected by centrifugation and resuspended in TMET (10 mM Tris-HC1, pH8, 2 mM MgCl, 1 mM ethylenediaminetetracetate (EDTA), 0.1 mM phenylmethylsulfonyl floride (PMSF), 0.1mM dithiothreitol (DTT), 1% Triton-X100R - about 50 ul/$10^6$ cells) at O°. The sample was centrifuged at 10,000 × g for 1 min, the pellet was discarded and the supernatant was brought to 1% sodium dodecylsulfate (SDS). Some of this sample was saved for protein assay by the (BCA) method Pierced Chemical Co., Rockford, Illinois). The remainder of the sample was made 0.1 M in B-mercaptoethanol (B-ME), heated at 95° for 5 min and stored at -20°.

For quantitation of ras protein, 1-10 ug of Triton-solubilized protein was subjected to SDS-PAGE and transferred to nitrocellulose substantially as described by Gross et al., Mol. Cell Biol. 5:1015 (1985). After transfer, the filter was rinsed in water, air-dried rehydrated in water and blocked by incubation for 30 min in PBS with 0.5% gelatin (autoclaved) at 37°. All subsequent steps were carried out in PBS with 0.2% gelatin and 0.25% Triton-X100 (PGT) at 37°. Filters were rinsed in PGT, incubated with ras specific antibody (40ug/ml) for 0.5 hr, washed 3× for 5 min in PGT, incubated with 125I-protein A (0.2 uCi/ml; 47 mCi/mg) for 0.5 hr, washed 4× for 5 min in PGT, dried and exposed to x-ray film with an intensifying screen at -70°.

## F. Focus assays:

5 × 10⁵ NIH 3T3 cells were added together with 10³, 10² or 10 cells of the line to be tested into 100 mm dishes in 10 ml of DMEM/10% Nu-serum. On the following day the serum concentration was decreased to 5% and the media was changed every 3-4 days thereafter. Foci were counted on days 12 and 18. To monitor viability and attachment of the various cell lines after trypsinization, 2 × 10⁵ cells of each line were added in a duplicate to 10 cm dishes in 10 ml of DMEM/10% Nu-serum. The cells were trypsinized and counted the next day. The numbers differed as much as +/- 15% from the mean.

## G. Soft agar assays:

Cells were trypsinized, washed, resuspended in DMEM/10% calf serum and diluted to 10⁵, 3.3 × 10⁴ and 1.1 × 10⁴ and 1.1 × 10.⁴ cells/ml in the same medium. 0.4 ml of cells was mixed with 0.6 ml of 0.5% agar in DMEM/10% serum. Duplicate 0.2 ml samples of this suspension were added to a 0.2 ml performed base layer of 0.5% agar-DMEM/10% serum in a 24 well plate. After 5 min at room temperature the cells were incubated at 37° in 5% CO₂ for 1 week. Colonies were strained with 0.2 ml of 0.05% p-iodonitrotetrazolium violet in H₂O and counted on an Omnicon image analysis system. Cell viability was monitored as in the focus assays. The variation in cell number was +/- 8% from the mean.

## H. tPA assays:

tPA activity was quantitated spectrophotometrically relative to a purified tPA standard (American Diagnostica, Greenwich, Connecticut) using the substrate 5-2251 (d-val-L-leu-L-lys-p-nitroanilide) (Kabi Vitrum, Stockholm, Sweden).

## 1. Transfection with H-ras and T24

NIH 3T3 cells were transfected with varying amounts of the ras vectors in supercoil form, unless otherwise noted, and were maintained in 5% Nu-serumR. Transformed cells appeared as foci of overgrowth in the monolayers at 7-12 days were counted at 15-20 days. The results of three experiments are shown in Table 1, below. In these experiments, the H-ras plasmids were 50-150 fold less efficient in focus formation than the T24 plasmids. Foci first appeared at 7-8 days with T24 plasmids and at 10-12 days with H-ras plasmids. Individual foci were trypsinized in cloning cylinders, expanded in medium containing 5% Nu-serumR and then subcloned in the same media.

The cloned foci were characterized with respect to their ras DNA, RNA and protein content and their focus-forming efficiency in mixed culture with NIH 3T3 cells. To date, we have analyzed four clones transformed by the mutant ras plasmid pMERT24 and four clones transformed by the normal H-ras plasmid pMERcHras. The copy number and organization of the transfected ras mini-genes was examined by Southern blot analysis. The number of potentially functional ras genes was estimated by cleavage at restriction sites bounding the transcription unit (BamHI plus EcoRI) and comparison to the parent plasmid.

Clones transformed by the mutant ras mini-gene contained 2-3 intact copies per 2N genome equivalents of DNA. In contrast, clones transformed by the normal ras mini-gene, contained 50-740 copies. The results for all of the clones are summarized in Table II, below. The organization of the ras sequences in the pMERcHras clones was further examined by cleavage with restriction enzymes having single or no sites in the transfecting plasmid. Several large fragments were observed with "noncutters" indicating that the vector was integrated into genomic or carrier DNA. In digests with "single cutters", the predominant fragment comigrated with the linearized vector. Together, these results indicate that the H-ras plasmid is integrated in tandem arrays of undetermined length (as few as two units) at multiple sites in carrier or genomic DNA. Tandem arrays of exogenously introduced DNA have been observed in the generation of transgenic mice and it was suggested that these structures formed by homologous recombination inside the transfected cell. However, the tandem ras sequences in out transformants probably result from resolution of multimeric or concatenated molecules in our plasmid preparations because these tandem structures were not observed in cells transformed with linear plasmids.

The expression of the human ras mini-genes in these transformants was examined at both the level of RNA and protein. As predicted from the intact transcription unit observed above, a prominent 1.5 kb RNA was observed by Northern blot analysis in the normal ras transformants. A similar transcript was detected at a much lower level in the T24 clones. Endogenous mouse H-ras transcripts were not observed, probably because of their low level and the reduced homology between the human mini-gene probe and mouse H-ras sequence. The levels of human ras RNA on Northern blots were quantitated relative to known amounts of ras plasmid DNA (Table II). The RNA levels in all of the multi-copy H-ras transformants are relatively high; but they are not always proportional to the DNA copy number, particularly in cells with a very high number of genes. Thus, at least some of the mini-genes are not transcriptionally equivalent in these different clones.

To measure ras protein levels, cells were lysed with 1% Triton X-100 and nuclei were removed by centrifuguration; essentially all of the p21 was in the supernatant. The p21 was quantitated relative to pure recombinant p21 (Gross et al., above) by immunoblot analysis. The level of p21 varied 20-fold among these cell lines and correlated well with the level of ras RNA (Table II). Thus, the translational efficiency of ras RNA is about equivalent in all of these cell lines.

We are characterizing some of the properties of these ras transformed clones in culture. Of particular interest are the genomic stability of the multicopy transfectants and the growth conditions which might select for the most efficient overproducers of p21 among the H-ras transformed clones. Initially, we have tested some of the transformed cell lines for their ability to reproduce the focus phenotype that was the basis for their isolation. Thus, $10^2$ or $10^3$ cells were plated together with $2 \times 10^5$ NIH 3T3 cells and the cultures were maintained in 5% Nu-serum. As in the original transfections, foci appeared most rapidly with the T24 clone. Surprisingly, the focus efficiency differed among the clones. H-ras-2.1 and T24-6 were about equally efficient, whereas a second H-ras clone, 15-2, yielded one-fourth as many foci. The apparent lower activity of 15-2 may result from the longer time for the appearance of these foci and the concomitant decay of the NIH 3T3 monolayer or from cellular heterogeneity in the 15-2 cell population. We have examined subclones of some of the cell lines found flat revertants, at low frequency, which lacked transfected ras sequences. Randomly selected transformed subclones had the same copy number as the parental clones. Cells transformed by the focus assay will grow in soft agar. For example clones 2-1 and 6 were positive in the soft agar assay and clones 3T3 and 5G5 were negative.

The high levels of p21 in the H-ras transformed Cells agrees with previous reports of transformation of rodent fibroblasts by overexpression of normal ras genes. Overexpression of p21 results from the acquisition of multiple gene copies. This observation, coupled with the low transformation efficiency of pMERcHras relative to pMERT24, indicates that focus formation with H-ras plasmids identifies rare high copy transfectants within the population of transfected cells. Initial results obtained with the doubly-selectable vectors pRas-neo and pT24-neo support this conclusion. Using these plasmids, we compared the relative efficiencies of focus formation and G418 resistance in the same transfection experiments. We found that these vectors produce G418 resistant colonies with about equal efficiency but that pRas-neo was about 200-fold less efficient than pT24-neo in focus formation. (Table I). For further characterization, random foci and G418-resistant colonies were picked from both sets of transfections and were expanded under G418 resistance. Both foci and G418-resistant clones obtained with pT24-neo contained a low number of intact ras + neo transcription units - 2-7 copies per 2N genome equivalent (Table III). Also, each of the pT24-neo G418-resistant clones gave foci in mixed culture with NIH 3T3 cells. We conclude that expression of one or a few integrated copies of pT24-neo can confer G418 resistance and produce foci. In contrast, for pRas-neo-derived cell lines, randomly selected foci had a higher average number of intact transcription units than the G418-resistant clones. In these foci, the copy number varied from 20 to 75 per 2N genome whereas in the G418-resistant clones, the copy number ranged from 2 to 35 (This latter value is higher than in the pT24-neo G418 resistant clones, probably because of the 5-50 fold greater DNA concentration in the pRas-neo transfection) (Table III). None of eight pRas-neo clones initially selected for G418 resistance formed foci in mixed culture with NIH 3T3 cells although the morphology of most of these clones differed from that of the parent cells. We therefore conclude that the H-ras foci derive from that subpopulation of cells having a high copy number of the transfected ras gene.

Finally, we have considered the possibility that cells containing multiple copies of H-ras are transformed because one or more of these genes mutated to a transforming ras variant. Indirect arguments against such a mutation are the high frequency of H-ras transformation relative to expected mutation rates and the strict correlation between high levels of p21 and transformation. However, to address this possibility directly, we transfected NIH 3T3 cells with genomic DNA from several H-ras and T24-ras transformed cell lines. DNAs from cells transformed by T24-ras genes all gave foci whereas none were observed with DNA from H-ras transformed cells (Table IV). Since the T24- and A5-1 T24-ras transformants have only one or two copies of an intact transforming ras gene, it is unlikely that the H-ras transformants contain even a single mutant ras gene.

The results we have described demonstrate that the H-ras foci result from overexpression of the H-ras protein. Thus, the high copy number of the transfected plasmid is selected because it can result in elevated protein expression. To address this point more directly, we have compared the H-ras protein levels in the pRas-neo foci with those in the G418-resistant clones. The highest level of p21 in a G418 resistant clonewhich lacked focus activity was 0.5 ng/ug protein and the lowest level in a focus was 2 ng/ug (Table III). Growing NIH 3T3 cells contain about 0.1 ng of total p21 (H-, K- and N-ras proteins) per ug of protein. Thus, transformation strictly correlates with a high-level accumulation of p21.

## 2. Transfections with H-ras and a Second Nonselected Gene.

In co-transfection experiments, a tPA mini-gene was introduced on the plasmid tPA-25. The bacterial plasmid pUC18 was also used as a nonselected DNA sequence. Vectors carrying the H-ras mini-gene, together with either tPA or neo transcriptional cassettes, were created to examine the linked introduction of a nonselected gene. The ras-neo vectors were described above. In the ras-tPA vectors, the tPA cDNA coding sequence was inserted between either the SV-40 early (pRas-tPA) or the RSV-LTR (pRas-RstPA) promoter and the bovine growth hormone polyadenylation signal. These tPA transcription cassettes were placed downstream of ras in pMERcHras in the same transcriptional orientation as ras.

The tPA-25 and pMERcHras vectors were co-transfected at different ratios in the presence of carrier DNA. Only a few foci were obtained in these transfections. As a preliminary screen for secretion of tPA, the foci were sequestered in cloning cylinders and incubated overnight with fresh media which was assayed for TPA activity.

Only the most rapidly appearing foci had significant activity and our futher characterization was limited to these clones. These clones were expanded into mass culture without subcloning and were analyzed for ras and tPA DNA and RNA sequences. Each contained more than 100 potentially functional ras mini-genes and even higher levels of tPA sequence. The levels of ras and tPA RNA were measured by Northern blot analysis relative to plasmid DNA standards. The RNA/DNA ratio for tPA was more than 10-fold lower than for ras. We do not know whether this low ratio for tPA results from inefficient transcription or instability of the tPA mRNA. However, in both clones, the tPA copy number was 5-10 times higher than ras and a low RNA/DNA ratio was consistently observed for ras or tPA in cells having a high copy number of the respective gene (note clones 14.2, 15.2, B1-5, B2A and 38f2 in Table II).

To compare the levels of tPA produced on a per cell basis with other cell lines, two of these clones, $B_1$ and $B_2$, were first subcloned. To assay for tPA production, cells were plated in duplicate at subconfluent densities and incubated two days with standard media. Medium and a cellular extract from one dish were assayed for tPA activity while the cell number was determined from the second dish. The results for a subclone of $B_2$ which contains about 150 copies of the tPA minigene are shown in Table II. These clones secrete about 50 fold less tPA than an optimized chinese hamster over cell line carrying about 400 copies of an amplified tPA mini-gene. The absence of tPA activity in the cellular extract indicates that tPA does not accumulate intracellularly.

In the above co-transfections, the molecular ratio of the tPA and ras plasmids was 2-4 to 1 and in each cell line, the copy number of tPA was greater than that of ras. To further examine the effect of plasmid ratios in cotransfections, pRAS-neo was co-transfected with the bacterial plasmid pUC-18 at ratios between 1:2 and 1:100. Several individual foci from each of these transfections were isolated and the levels of ras and pUC 18 DNA sequence were quantitated by blot analysis. In general, the sequence ratio in each group of transfectants paralleled, but was not identical to, the relative input of the two plasmids. Thus, the copy number of a non-linked gene an be varied in co-transfected cells by altering its ratio to the ras gene. We again point out, however, that very high gene numbers did not result in a corresponding elevation of steady-state RNA levels (Table II). We do not know whether this reduced efficiency at high copy member reflects limitations imposed by the cells or by our methodology. It is known, for example, that certain cell lines are incapable of expressing large amounts of certain proteins. What is important, however, is that by using the method of the invention, a large number of genes were integrated in a single transfection and selection cycle.

A tPA minigene driven by an SV40 or RSV promotor was inserted into the pMERcHras expression vector in the same transcription orientation (see above). These vectors were transfected into NIH 3T3 cells and resultant foci were screened for tPA secretion. Several of the more rapidly growing foci showed significant activity and were subcloned and expanded. We analyzed several of these lines for ras and tPA DNA, RNA and protein as described above. These results are summarized in Table II. Two of these lines secrete much higher levels of tPA than observed for the cotransformed cells although still lower levels than the optimized CHO cell line.

Table I - Transformation Efficiency

| Plasmid | Amount (µg) | Foci | G418 colonies | Efficiency Foci | G418[r] |
|---|---|---|---|---|---|
| **Exp't 1** | | | | | |
| pMERcHras | 0.1 | 40 | | 400 | |
| pMERT24 | 0.02 | 1200 | | 60,000 | |
| | | a | | | |
| **Exp't 2** | | | | | |
| pMERcHras | 5 | 240 | | 48 | |
| pMERT24 | 0.1 | 225 | | 2300 | |
| **Exp't 3** | | | | | |
| pRas-neo | 0.5 | 6 | 620 | 12 | 1200 |
| pT24-neo | 0.1 | 300 | 300 | 3000 | 3000 |

a The indicated amount of plasmid was precipitated together witn 10ug of NIH 3T3 DNA and added to aoout $10^6$ NIH 3T3 cells in a 60 mm culture dish.

b Total numoer of foci or colonies per transfected disn. Tnis value is an average from two transfected disnes in Exp't 1; in tne otner assays only one dish was transfected. Control transfections witn only 3T3 DNA gave no foci.

c The numoer of foci or G418[r] colonies/ugDNA/$10^6$ cells. DNA transfections in Exp't 1 were carried out as described by Wigler et al and in Exp't 2 and 3 as descrioed oy Copeland et al. The 10-fold nigher transformation efficiency in Exp't 1 may nave resulted from tnis procedural difference.

Table II - Summary of the Expression of ras and TPA

| | ras | | | | | TPA | | | | |
|---|---|---|---|---|---|---|---|---|---|---|
| Cell line | DNA[a] (copy #) | RNA[b] (pg/ug) | Protein[c] (ng/ug) | RNA/DNA[d] | Protein[e]/RNA | DNA[a] (copy #) | RNA[b] pg/ug | Protein[f] (pg/cell/day) | RNA/DNA[d] | Protein[g]/RNA |
| H-ras | | | | | | | | | | |
| 2.1 | 50 | 5 | 8.0 | 0.1 | 1.6 | | | | | |
| 14.2 | 210 | 2.5 | 2.7 | 0.01 | 1.1 | | | | | |
| 15.2 | 740 | 3 | 2.0 | 0.004 | 1.7 | | | | | |
| 18 | 40 | 2 | 3.3 | 0.08 | .1.7 | | | | | |
| T24 ras | | | | | | | | | | |
| 6 | 2 | 0.1 | 0.2 | 0.05[h] | 2[h] | | | | | |
| R1 | 2 | | 0.3 | | | | | | | |
| R2 | 3 | | 0.4 | | | | | | | |
| R3 | 2 | | 0.2 | | | | | | | |
| 3T3 | - | - | 0.1 | | | | | | | |
| TPA/wt ras cotransf. | | | | | | | | | | |
| A1 | 60 | | | | | 350 | | | | |
| 91-5 | 50 | 2.5 | 3.3 | 0.05 | 1.3 | 350 | 1.0 | | 0.003 | |
| 92A | 40 | 1.5 | 2.0 | 0.04 | 1.3 | 150 | 0.5 | 0.15 | 0.003 | 0.03 |
| TPA/wt ras linked transf. | | | | | | | | | | |
| TPA (SV40) | | | | | | | | | | |
| 13f3 | 40 | 1.5 | 7 | 0.04 | 4.7 | 40 | 0.9 | 1.1 | 0.03 | 1.2 |
| 13f8 | 80 | 2 | 7 | 0.02 | 3.5 | 80 | 1.0 | 0.28 | 0.01 | 0.3 |
| 13f9 | 60 | 1.5 | 3 | 0.03 | 2.0 | 60 | 0.9 | 0.60 | 0.02 | 0.7 |
| 13f13 | 60 | 2 | 4 | 0.03 | 2.0 | 60 | 0.8 | 0.14 | 0.01 | 0.2 |
| TPA (RSV) | | | | | | | | | | |
| 38f2 | 370 | 2.5 | 6 | 0.007 | 2.4 | 370 | 0.9 | 0.19 | 0.002 | 0.2 |

[a] Copy number of the intact transcription unit per 2N genome equivalents estimated relative to known amounts of plasmid in DNA blots.

[b] pg of ras or TPA RNA per ug of total cellular RNA. The amounts of ras and TPA RNA were estimated from Northern blots (1.5 kb for ras and 4.0 kb for TPA) relative to known amounts of plasmid DNA slot-blotted onto a parallel filter.

[c] ng of p21 per ug of total Triton X-100 soluble protein as measured from immunoblots with pure recombinant H-ras p21 as a standard.

[d] Ratio of (pg ras RNA/mg total RNA) to (DNA copies/2N) or corresponding values for TPA.

[e] Ratio of (ng p21/ug protein) to (pg ras RNA/mg total RNA).

[f] The total amount of TPA activity in the medium, relative to known amounts of purified TPA, after a two-day incubation divided by the cell number and days of incubation.

[g] Ratio of TPA activity (pg/cell/day) to (pg TPA RNA/mg total RNA).

[h] These ratios are very approximate because of the very low levels of ras RNA and protein and because of the probable significant contribution of endogenous ras proteins.

Table III

## Summary of the Expression of ras in ras/neo Transformants

| Cell Line | | DNA [c] (copies/2N) | p21 [d] (ng/ug) |
|---|---|---|---|
| T24 ras/neo transformants [a] | | | |
| foci: | F1 | 6 | 0.3 |
| | F2 | 7 | |
| G418[r]: | G1 | 2 | 0.2 |
| | G2 | 2 | |
| | | | |
| H-ras/neo transformants [b] | | | |
| foci: | 5F1 | 75 | |
| | 5F2 | 75 | 2.0 |
| | | | |
| G418[r]: | 5G2 | 30 | 0.2 |
| | 5G4 | 3 | 0.2 |
| | 5G5 | 30 | 0.5 |
| | 0.5G4 | 15 | 0.2 |
| | 0.5G5 | 2 | 0.2 |
| | 0.5G6 | 30 | 0.3 |

a   $10^6$ 3T3 cells were transfected witn 0.1 ug of pT24-neo plus 10 ug 3T3 DNA.
b   $10^6$ 3T3 cells were transfected witn 0.5 or 5ug of pRas-neo plus 10ug 3T3 DNA. Clones or foci designated by 5G- or 5F- were from tne 5ug transfection and tnose lanelled 0.5 G- were from tne 0.5ug transfection.
c   See footnote a in Tanle II.
d   See footnote c in Taole II.

Table IV - Transfections with Genomic DNA

| Cell Line | ras Copy # | Foci[d] | G418[r] [d] Colonies | Efficiency[e] Foci | Efficiency[e] G418[r] |
|---|---|---|---|---|---|
| H-ras 2.1[a] | 50 | 0 | | 0 | |
| Ras-neo 5F2[b] | 75 | 0 | 117 | 0 | 25 |
| T24-6[a] | 2 | 7 | 0 | 0.2 | - |
| T24-neo F1[b] | 6 | 10 | 10 | 0.3 | 2.2 |
| A5-1[c] | 2 | 61 | | 1.5 | |
| 3T3 | - | 0 | | 0 | |

[a] Described in Table II

[b] Described in Table III

[c] NIH-3T3 cell transformed by the genomic T24 ras gene. This cell line is a secondary transformant of NIH-3T3 derived from transformation by genomic DNA from the T24 bladder carcinoma cell line.

[d] 20ug of genomic DNA from the indicated cell line was precipitated and added in duplicate to $10^6$ NIH-3T3 cells in a 60 mm culture dish. One day after transfection, each dish was split into three 100 mm dishes. To measure G418 resistance, 1/9th of the culture, removed during the cell split, was grown in 600 ug/ml G418. Cells transfected with T24-6 DNA, which does not carry a neo gene, were used as a negative control. Foci and G418[r] colonies were counted at 23 days and are given as total numbers for all dishes.

[e] Number of foci or G418[r] colonies per ug of DNA.

The above description and examples fully disclose this invention including the preferred embodiments thereof. The invention, however, is not limited to the constructions disclosed herein but, rather, encompasses all modifications and variations coming with the scope of the claims which follow.

**Claims**

1. A method for producing a recombinant mammalian cell line having multiple copies of a gene of interest which comprises: (1) cotransfecting a mammalian cell, which can be transformed by increased expression of a protooncogene, with the gene of interest and with the protooncogene and (2) selecting transfectants which are transformed and which express the gene of interest.

2. The method of claim 1 in which the protooncogene is a normal H-ras, N-ras or K-ras oncogene.

3. The method of claim 1 in which the protooncogene is a normal H-ras gene.

4. The method of claim 2 in which the cell to be transfected is a contact inhibited rat fibroblast cell.

5. The method of claim 3 in which the cell to be transfected is a NIH 3T3 cell.

6. A recombinant mammalian cell line prepared by the method of claim 1.

7. A process for preparing a gene product of interest which comprises culturing the cell line of claim 6 and isolating and purifying the gene product of interest therefrom.

| | **DOCUMENTS CONSIDERED TO BE RELEVANT** | | EP 87308646.6 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | MOLECULAR AND CELLULAR BIOLOGY, vol. 5, no. 10, October 1985 (Washington DC.) S. PULCIANI et al. "ras Gene Amplification and Malignant Transformation" pages 2836-2841 * Totality * -- | 1-7 | C 12 N 15/00 C 12 N 5/00 |
| D,A | NATURE, vol. 297, no. 5866, June 10, 1982 (New York, London) E.H. CHANG et al. "Tumorigenic transformation of mammalian cells induced by a normal human gene homologous to the oncogene of Harvey murine sarcoma virus" pages 479-483 * Totality * -- | 1-7 | |
| D,A | US - A - 4 399 216 (AXEl et al.) * Abstract * -- | 1 | **TECHNICAL FIELDS SEARCHED (Int. Cl.4)** C 12 N |
| D,A | WO - A1 - 82/00 158 (THE SALK IN-STITUTE FOR BIOLOGICAL STUDIES) * Claim 1 * -- | 1 | |

The present search report has been drawn up for all claims

| Place of search | Date of completion of the search | Examiner |
|---|---|---|
| VIENNA | 05-01-1988 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82

European Patent
Office

**EUROPEAN SEARCH REPORT**

Application number

| | **DOCUMENTS CONSIDERED TO BE RELEVANT** | | EP 87308646.6 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | ANNUAL REVIEW OF GENETICS, vol. 18, 1984, (Palo Alto, USA) <br><br> H.E. VARMUS "The Molecular Genetics of Cellular Oncogenes" pages 553-612 <br><br> * Totality * <br><br> ---- | | |
| | | | TECHNICAL FIELDS SEARCHED (Int. Cl.4) |

The present search report has been drawn up for all claims

| Place of search | Date of completion of the search | Examiner |
|---|---|---|
| VIENNA | 05-01-1988 | WOLF |